# EUROPÄISCHE PATENTSCHRIFT

(11) **EP 2 618 854 B1**
(45) Veröffentlichungstag und Bekanntmachung des Hinweises auf die Patenterteilung: **14.06.2017**
(21) Anmeldenummer: 11758446.6
(22) Anmeldetag: 14.09.2011
(51) Int. Cl.: A61L 2/26

(54) **STERILBEHÄLTER**
STERILE CONTAINER
CONTENANT STÉRILE

(30) Priorität: 20.09.2010 DE 102010037659
(43) Veröffentlichungstag der Anmeldung: 31.07.2013
(60) Teilanmeldung: 17154245.9
(73) Patentinhaber: Aesculap AG, 78532 Tuttlingen (DE)
(72) Erfinder: GLEICHAUF, Wilhelm, 78532 Tuttlingen-Möhringen (DE); JAKAB, Mariana, 78532 Tuttlingen (DE); SCHUSTER, Stefan, 78048 Villingen-Schwenningen (DE); THOMAS, Stefan, 78532 Tuttlingen (DE); GRAY-DREIZLER, John, 78628 Rottweil (DE)
(74) Vertreter: Hoeger, Stellrecht & Partner Patentanwälte mbB
(86) Internationale Anmeldenummer: PCT/EP2011/065960
(87) Internationale Veröffentlichungsnummer: WO 2012/038314

(56) Entgegenhaltungen:
- EP-A1- 0 080 203
- EP-A2- 0 274 706
- WO-A1-02/40063
- DE-A1-102004 028 040
- US-A- 3 531 013
- US-A- 4 034 889
- US-A- 4 412 630
- US-A1- 2006 151 509

## Beschreibung

Die Erfindung betrifft einen Sterilbehälter zur Sterilisation chirurgischer Instrumente, umfassend eine Bodenwand, eine Seitenwand sowie einen Deckel, der in einer geschlossenen Stellung des Sterilbehälters mit einem Deckelrand einen an einer der Bodenwand abgewandten Seite der Seitenwand angeordneten freien Rand übergreift.

In derartigen Sterilbehältern werden die zur Sterilisation bestimmten chirurgischen Instrumente platziert, und der Deckel wird zum Schließen des Sterilbehälters über die Seitenwand gestülpt. Mittels Befestigungselementen, beispielsweise in Form von Schnallen, wird der Deckel an der Seitenwand so festgelegt, dass zwischen der Seitenwand und dem Deckel im geschlossenen Zustand des Sterilbehälters eine möglichst gute Abdichtung erzielt werden kann. Dies ist von Bedeutung, da ein Gasaustausch zwischen dem Behälterinnenraum und der Atmosphäre allein durch zwischen in die Seitenwand und/oder in den Deckel eingelassene Filterelemente erfolgen soll. Dennoch kann es in der Praxis vorkommen, dass eine Bedienperson am Deckelrand des geschlossenen Behälters unbeabsichtigterweise so anstößt oder diesen untergreift, dass ein Gasaustausch zwischen dem Behälterinnenraum und der Atmosphäre auch durch einen Spalt zwischen dem freien Rand und dem Deckelrand hindurch möglich ist. Dies wirkt sich aufgrund fehlender Filterung des ausgetauschten Gases negativ auf das Sterilisationsergebnis aus.

Die US 2006/0151509 A1 beschreibt einen Sterilbehälter zur Aufnahme und sterilen Aufbewahrung von chirurgischem Besteck oder Material, der einen Aufnahmebehälter und einen Deckel umfasst. Der Deckel weist eine Aufsetzhilfe zum Aufsetzen des Deckels auf den Aufnahmebehälter auf, die mehrere Aufsetzhilfeelemente umfasst, welche in einer Verschlussstellung des Deckels jeweils mindestens eine Behälterwand mindestens teilweise überlappen.

Die US 4,412,630 beschreibt einen nicht für medizinische Sterilisationszwecke einsetzbaren Kunststoffbehälter aus einem elastisch verformbaren Material. Der Behälter umfasst eine Vorrichtung, mit der ein Dichtungselement eines Behälterdeckels relativ zu einem Dichtungselement einer Behälterwanne ausgerichtet werden kann. Die Vorrichtung umfasst mindestens eine Ausrichtungslasche, die mit mindestens einem Ausrichtungsspalt zusammenwirkt, um die Dichtungselemente relativ zueinander auszurichten.

Ein weiterer, nicht zur Sterilisation medizinischer Instrumente geeigneter Behälter aus einem elastisch verformbaren Kunststoffmaterial ist in der US 4,034,889 beschrieben. Ein Behälterdeckel weist abwinkelbare Außenränder auf, die zum Verschließen des Behälters um Filmscharniere relativ zu einem Deckelabschnitt abgewinkelt und durch Verrastung mit einer Behälterwand verbunden werden können.

Auch ein in der US 3,531,013 beschriebener Behälter besteht aus einem elastisch verformbaren Material und ist nicht zur Sterilisation chirurgischer Instrumente geeignet. Ein Behälterdeckel weist eine Aufnahme auf, in die ein freier Rand einer Behälterwand, der wellenförmig ausgestaltet ist, in einer Verschlussstellung des Deckels eingreifen kann, so dass sich der Deckel und die Behälterwand über gegenseitige Hinterschnitte sichern.

Die EP 0 274 706 A2 beschreibt einen Einwegbehälter für Krankenhausabfälle, der mittels zweier verschiedener Verschlüsse einerseits vorläufig und andererseits endgültig verschließbar ist. Der endgültige Verschluss ist nicht ohne Zerstörung des Verschlusses wieder zu öffnen.

Ein medizinischer Sterilisierbehälter ist in der EP 0 080 203 A1 beschrieben, der aus einem Unterteil und Deckel besteht. Ferner ist ein Zwischendeckel vorgesehen, der dichtend auf einen freien Rand des Unterteils aufgesetzt ist. Der freie Rand ist in Richtung des Behälterinnenraums eingezogen.

Ein weiterer Sterilbehälter ist in der DE 10 2004 028 040 A1 beschrieben.

Die WO 02/40063 A1 beschreibt Mehrzweckverpackungen für sterilisierte oder zu sterilisierende Gegenstände.

Aufgabe der vorliegenden Erfindung ist es, einen gattungsgemäßen Sterilbehälter bereitzustellen, der eine bessere Handhabbarkeit unter Erzielung eines möglichst guten Sterilisationsergebnisses aufweist.

Diese Aufgabe wird erfindungsgemäß durch einen Sterilbehälter mit den Merkmalen von Anspruch 1 gelöst.

Der erfindungsgemäße Sterilbehälter umfasst mindestens ein Vorsprungelement an der Seitenwand, das zwischen dem freien Rand und der Bodenwand angeordnet ist, also zwischen der Oberseite und der Unterseite der Seitenwand, bezogen auf eine Gebrauchsstellung des Sterilbehälters, in welcher dieser mit der Bodenwand auf einer Aufstellfläche ruht. Das Vorsprungelement ist von der Bodenwand weniger weit beabstandet als der den freien Rand übergreifende Deckelrand, also bei geschlossenem Sterilbehälter unterhalb desselben angeordnet. Zusätzlich springt es außenseitig am Sterilbehälter von der Seitenwand mindestens so weit vor, dass es bis zum Deckelrand reicht. Auf diese Weise ist die Gefahr verringerbar, dass eine Bedienperson unbeabsichtigterweise so an dem Deckelrand anstößt oder diesen untergreift, dass sich der Deckel, wenn auch hier nur in geringem Umfang, so vom Sterilbehälter im Übrigen löst, dass ein Gasaustausch zwischen dem Deckel und dem freien Rand hindurch ermöglicht wird. Dies sichert zum einen unmittelbar ein gutes Sterilisationsergebnis, was zum anderen Einfluss auf die Handhabung des Sterilbehälters hat, denn bei Vorliegen eines unzureichenden Sterilisationsergebnisses ist es für die Bedienperson erforderlich, die chirurgischen Instrumente erneut zu sterilisieren.

Zudem ragt die Seitenwand beim erfindungsgemäßen Sterilbehälter in einem Wandbereich oberhalb des mindestens einen Vorsprungelementes nicht in Richtung der Sterilbehältermitte über die Seitenwand in einem Wandbereich unterhalb des mindestens einen Vorsprungelementes hervor. Dadurch verschmälert sich der Behälterinnenraum im Wandbereich zwischen dem mindestens einen Vorsprungelement und dem freien Rand der Seitenwand nicht. Infolgedessen ist die Gefahr erheblich verringerbar, dass eine Bedienperson, wenn sie die chirurgischen Instrumente dem Behälterinnenraum entnimmt, am oberen Wandbereich zwischen dem mindestens einen Vorsprungelement und dem freien Rand anstößt sowie gar an diesem hängen bleibt. Dies wirkt sich unmittelbar vorteilhaft auf die Handhabbarkeit des Sterilbehälters aus. Ferner ist die Wahrscheinlichkeit erheblich verringert, dass ein Instrument der Bedienperson durch Anstoßen oder Hängenbleiben entfällt und schlimmstenfalls gar zu Boden fällt, was dessen Sterilität beeinträchtigt und einen neuen Sterilisationsvorgang erforderlich macht. Sind die chirurgischen Instrumente im Behälterinnenraum in einem Siebkorb untergebracht, ist die Gefahr weitgehend vermeidbar, dass ein Instrument aus dem Siebkorb herausgeschleudert und kontaminiert wird. Dies sichert die Sterilität der Instrumente ebenfalls.

Günstig ist es, wenn die Seitenwand im Wandbereich zwischen dem mindestens einen Vorsprungelement und dem freien Rand in Richtung der Außenseite über die Seitenwand im Wandbereich zwischen dem mindestens einen Vorsprungelement und der Bodenwand hervorragt. Dadurch erweitert sich der Behälterinnenraum im oberen Wandbereich zwischen dem mindestens einen Vorsprungelement und dem freien Rand. Dies erleichtert es der Bedienperson, Instrumente in den Sterilbehälter einzuführen und aus diesem zu entnehmen.

Vorzugsweise springt mindestens ein Vorsprungelement, insbesondere alle Vorsprungelemente, über den Deckelrand hinaus in Richtung der Außenseite von der Seitenwand vor. Dadurch ist die Wahrscheinlichkeit, dass eine Bedienperson unbeabsichtigterweise am Deckelrand anstößt oder diesen untergreift, weiter verringerbar. Infolgedessen findet auch ein unerwünschter Gasaustausch zwischen dem Behälterinnenraum und der Atmosphäre zwischen dem Deckel und dem freien Rand hindurch mit geringerer Wahrscheinlichkeit statt.

Von Vorteil ist es, wenn mindestens ein Vorsprungelement den Deckelrand an einer der Seitenwand abgewandten Seite hintergreift. Dadurch ist die Wahrscheinlichkeit, dass eine Bedienperson am Deckelrand anstößt oder diesen untergreift und einen unerwünschten Gasaustausch zwischen dem Behälterinnenraum und der Atmosphäre verursacht, noch weiter verringerbar. Beispielsweise greift der Deckelrand in eine zwischen der Seitenwand und dem mindestens einen Vorsprungelement gebildete nutförmige Ausnehmung ein.

Günstig ist es, wenn mindestens ein Vorsprungelement, bevorzugt alle Vorsprungelemente, ein Anlageelement für den Deckelrand umfasst oder ausbildet, beispielsweise eine Anlagefläche, auf der der Deckelrand obenseitig aufliegen kann. Dadurch kann der Deckel relativ zum Sterilbehälter im Übrigen im geschlossenen Zustand eine besonders definierte Position einnehmen. Über das mindestens eine Vorsprungelement kann sich der Deckel an der Seitenwand so abstützen, dass der Deckel im geschlossenen Zustand mit einer stärkeren Zugspannung als bislang an dem Sterilbehälter im Übrigen fixiert werden kann. Dadurch kann auch eine höhere Dichtigkeit zwischen dem Deckel und dem freien Rand der Seitenwand erzielt werden.

Bei einer andersartigen vorteilhaften Ausführungsform des erfindungsgemäßen Sterilbehälters kann vorgesehen sein, dass der Deckelrand in der geschlossenen Stellung des Sterilbehälters relativ zu dem mindestens einen Vorsprungelement beabstandet ist. Dadurch kann beispielsweise ein während des Sterilisiervorgangs entstehender Überdruck im Sterilbehälter abgebaut werden, indem der Deckel infolge des Überdrucks etwas angehoben und/oder verformt und dadurch ein Nebenstrompfad zwischen der Seitenwand und dem Deckel freigegeben wird.

Die Seitenwand umfasst mindestens ein Vorsprungelement und günstigerweise alle Vorsprungelemente. Dies ermöglicht eine konstruktiv einfache Ausgestaltung des Sterilbehälters.

Eine noch einfachere konstruktive Ausgestaltung des Sterilbehälters wird dadurch ermöglicht, dass das mindestens eine von der Seitenwand umfasste Vorsprungelement einstückig mit der Seitenwand gebildet ist.

Das Vorsprungelement ist durch Umformen der Seitenwand gebildet, wobei es beispielsweise aus einer von der Seitenwand im Übrigen definierten Ebene in Richtung der Außenseite hervorspringt. Dies gibt z.B. die Möglichkeit, der Seitenwand eine steifere Konstruktion zu verleihen.

Bei einer nicht beanspruchten Ausführungsform ist das Vorsprungelement eine in der Seitenwand gebildete Sicke. Dadurch lässt sich das Vorsprungelement auf konstruktiv besonders einfache Weise mit der Seitenwand einstückig ausbilden und zudem eine wünschenswerte Versteifung der Seitenwand erzielen. Bei der Sicke kann es sich um eine Vollsicke oder um eine Halbsicke handeln, und die Sicke kann beispielsweise einen U-förmigen, einen rechteckförmigen, einen dreieckförmigen, einen wellenförmigen oder einen pulsförmigen Querschnitt aufweisen.

Bei einer andersartigen Ausführungsform des Sterilbehälters, bei der das Vorsprungelement einstückig mit der Seitenwand gebildet ist, kann das Vorsprungelement vorzugsweise durch Umbiegen der Seitenwand am freien Rand in Richtung der Außenseite gebildet sein. Dadurch lässt sich ebenfalls auf konstruktiv einfache Weise das Vorsprungelement unter Versteifung der Seitenwand bilden.

Bei einer nicht beanspruchten Ausführungsform eines Sterilbehälters ist es günstig, wenn mindestens ein Vorsprungelement, beispielsweise auch alle Vorsprungelemente, getrennt von der Seitenwand gebildet und an dieser festgelegt ist. Dies ermöglicht es etwa unter Einsatz unterschiedlicher Materialien zur Fertigung der Seitenwand und des mindestens einen Vorsprungelementes, diese bedarfsgerecht für den jeweiligen Einsatzzweck auszubilden. Dadurch können unter Umständen Kosteneinsparungen bei der Herstellung des Sterilbehälters und/oder eine robustere Ausgestaltung des Sterilbehälters erzielt werden. Das Vorsprungelement ist insbesondere außenseitig an der Seitenwand kraftschlüssig, formschlüssig und/oder stoffschlüssig festgelegt, beispielsweise durch Kleben, Nieten, Schrauben oder Schweißen. Denkbar ist der Einsatz einer Klemmverbindung, einer Rastverbindung oder einer Steckverbindung, um das Vorsprungelement an der Seitenwand festzulegen.

Es kann bei einer nicht beanspruchten Ausführungsform vorgesehen sein, dass das Vorsprungelement lösbar an der Seitenwand festgelegt ist.

Das Vorsprungelement ist bei einer nicht beanspruchten Ausführungsform günstigerweise ein insbesondere längserstrecktes Profil, beispielsweise ein L-Profil oder ein J-Profil, und es kann als Gitter-Profil oder als Lochblech-Profil ausgestaltet sein. Mittels eines Profiles, das außenseitig an der Seitenwand festgelegt ist, kann eine erhebliche Versteifung derselben erzielt werden

Bei einer andersartigen nicht beanspruchten Ausführungsform eines Sterilbehälters kann vorgesehen sein, dass mindestens ein Vorsprungelement stiftförmig ausgebildet ist, welcher Stift in seiner Längsrichtung nach außen von der Seitenwand vorspringt. Das stiftförmige Vorsprungelement kann von der Seitenwand umfasst und insbesondere einstückig mit dieser gebildet sein, oder es ist getrennt von dieser gebildet und an dieser festgelegt.

Vorzugsweise ist das mindestens eine Vorsprungelement sterilisierbar, um einen bestimmungsgemäßen Einsatz des Sterilbehälters zu ermöglichen.

Es ist vorgesehen, dass mindestens ein Vorsprungelement aus Metall gefertigt ist, beispielsweise aus Aluminium, Titan oder Edelstahl. Auch ein Einsatz einer Metalllegierung für das Vorsprungelement ist möglich.

Ferner kann vorgesehen sein, dass mindestens ein weiteres Vorsprungelement zumindest teilweise aus Kunststoff gefertigt ist und insbesondere insgesamt aus Kunststoff gefertigt ist, etwa aus einem Polyetheretherketon (PEEK), aus Polyphenylsulfon (PPSU) oder aus einem Silikonmaterial.

Das mindestens eine Vorsprungelement ist bevorzugt, zumindest teilweise, in einer parallel zur Bodenwand und/oder parallel zum Deckel verlaufenden Horizontalebene angeordnet. Ist mehr als ein Vorsprungelement vorhanden, können diese in unterschiedlichen Horizontalebenen angeordnet sein.

Vorzugsweise ist mindestens ein Vorsprungelement an einer Längsseite des Sterilbehälters angeordnet, wobei es sich günstigerweise längs der gesamten Längsseite erstreckt. Dadurch kann die Wahrscheinlichkeit, dass eine Bedienperson unbeabsichtigterweise den Deckelrand untergreift oder an diesem anstößt, weiter verringert werden.

In entsprechender Weise kann vorgesehen sein, dass mindestens ein Vorsprungelement an einer Querseite des Sterilbehälters angeordnet ist und sich speziell längs der gesamten Querseite des Sterilbehälters erstreckt. Auch in diesem Fall ist die Wahrscheinlichkeit verringerbar, dass eine Bedienperson unbeabsichtigterweise den Deckelrand untergreift.

Günstig ist es, wenn mindestens ein Vorsprungelement längs eines gesamten Umfanges oder im Wesentlichen längs eines gesamten Umfanges des Sterilbehälters verläuft. Das Vorsprungelement erstreckt sich bei dieser Ausführungsform ganz oder im Wesentlichen ganz längs der den Behälterinnenraum einfassenden Seitenwand. Dadurch ist der Deckelrand längs des gesamten oder im Wesentlichen gesamten Umfangs des Behälterinnenraums gegen ein unbeabsichtigtes Anstoßen oder Untergreifen geschützt.

Es kann auch vorgesehen sein, dass mindestens ein Vorsprungelement entlang einer zusammenhängenden Randlinie längs eines Umfanges des Sterilbehälters verläuft, beispielsweise dann, wenn über das Vorsprungelement hinaus an der Seitenwand Befestigungselemente zum Festlegen des Deckels am Sterilbehälter im Übrigen angeordnet sind.

Ferner kann vorgesehen sein, dass eine Mehrzahl von Vorsprungelementen, jeweils entlang einer zusammenhängenden Randlinie längs eines Umfangs des Sterilbehälters verlaufen und in Umfangsrichtung voneinander beabstandet sind. Beispielsweise können zwischen zwei voneinander beabstandeten Vorsprungelementen Befestigungselemente wie etwa Schnallen zur Befestigung des Deckels vorhanden sein.

Dem Sterilbehälter kann eine einfache konstruktive Ausgestaltung vorteilhafterweise dadurch verliehen werden, dass der Sterilbehälter genau ein Vorsprungelement umfasst. Insbesondere kann das genau eine Vorsprungelement bei einer nicht beanspruchten Ausführungsform eine an der Seitenwand gebildete Sicke sein, die ganz oder im Wesentlichen ganz längs des Umfanges des Behälters verläuft.

Bei einer andersartigen Ausführungsform des erfindungsgemäßen Sterilbehälters kann vorgesehen sein, dass der Sterilbehälter eine Mehrzahl von Vorsprungelementen umfasst.

In letzterem Fall kann die konstruktive Ausgestaltung des Sterilbehälters dadurch einfach gestaltet werden, dass alle Vorsprungelemente des Sterilbehälters identisch ausgebildet sind.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung einer ersten Ausführungsform eines Sterilbehälters im geöffneten Zustand;
- Figur 2:: eine Schnittansicht in einer Mittelquerebene des Sterilbehälters aus Figur 1 im geschlossenen Zustand und
- Figuren 3 bis 11:: Detailansichten einer zweiten bis einer zehnten bevorzugten Ausführungsform des Sterilbehälters in einer dem Detail A in Figur 2 entsprechenden Darstellung, wobei Figur 8 eine erfindungsgemäße Ausführungsform zeigt.

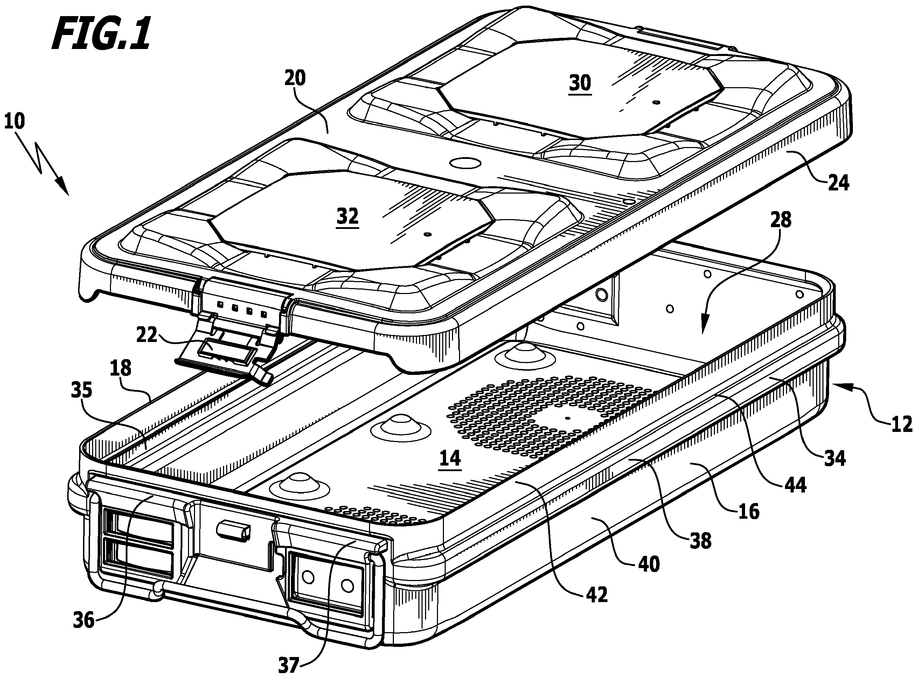

Eine erste bevorzugte Ausführungsform eines Sterilbehälters zur Sterilisation chirurgischer Instrumente, die lose oder in einem Siebkorb im Behälterinnenraum positionierbar sind, ist in Figur 1 im geöffneten Zustand perspektivisch dargestellt und dort insgesamt mit dem Bezugszeichen 10 belegt. Der Sterilbehälter 10 umfasst eine Wanne 12 mit einer Bodenwand 14 sowie einer umlaufenden Seitenwand 16, die obenseitig einen freien Rand 18 aufweist. "Obenseitig" bezieht sich vorliegend auf eine Gebrauchsstellung des Sterilbehälters 10, in welcher dieser mit der Bodenwand 14 auf einer Aufstellfläche aufliegt.

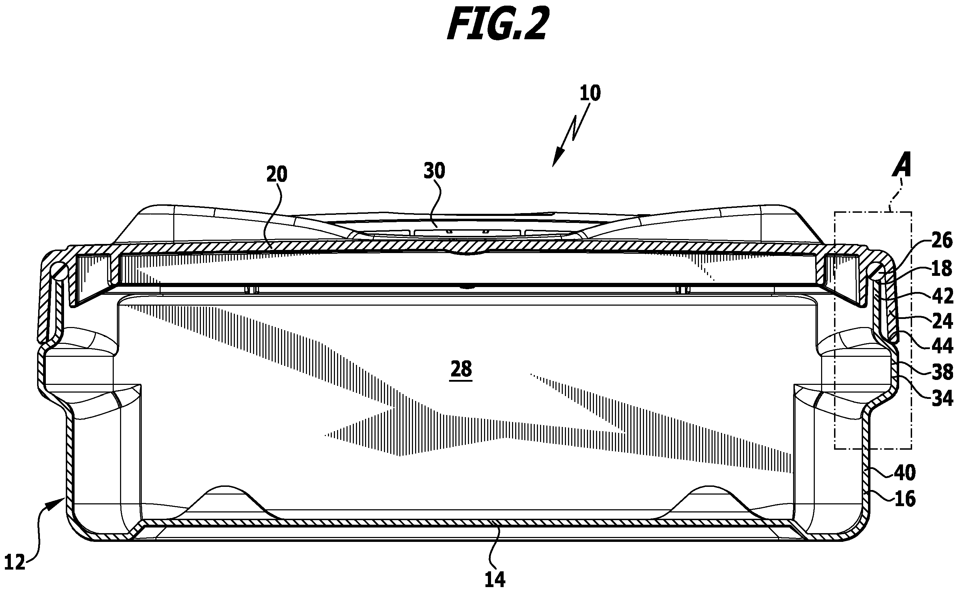

Ferner umfasst der Sterilbehälter 10 einen von einer geöffneten Stellung (Figur 1) in eine geschlossene Stellung (Figur 2) und umgekehrt überführbaren Deckel 20. In der geschlossenen Stellung kann der Deckel 20 mittels an sich bekannter Befestigungselemente in Form von Schnallen (nur eine Schnalle 22 ist gezeigt), an der Wanne 12 befestigt werden. Bei dem Deckel 20 handelt es sich um einen so genannten "Stülpdeckel", der den freien Rand 18 im geschlossenen Zustand des Sterilbehälters 10 mit einem Deckelrand 24 längs des gesamten Umlaufs der Seitenwand 16 übergreifen kann (Figur 2).

Ein Dichtelement 26 ist am Deckelrand 24 gehalten, das den Sterilbehälter 10 im geschlossenen Zustand gegen einen Gasaustausch zwischen einem Behälterinnenraum 28 und der Atmosphäre sichert, welcher Gasaustausch durch einen Spalt zwischen dem freien Rand 18 und dem Deckel 20 hindurch verläuft. Ein Gasaustausch zwischen dem Behälterinnenraum 28 und der Atmosphäre soll stattdessen nur durch in den Deckel 20 eingesetzte Filtereinrichtungen 30 und 32 hindurch stattfinden.

Der Sterilbehälter 10 weist eine Mehrzahl von an der Seitenwand 16 angeordneten Vorsprungelementen 34 bis 37 auf, von denen die Vorsprungelemente 34 und 35 jeweils an einer der Längsseiten des Sterilbehälters 10 an der Seitenwand 16 angeordnet sind. Die Vorsprungelemente 36 und 37 sind ebenfalls identisch ausgebildet und an einer Querseite des Sterilbehälters 10 an der Seitenwand 16 angeordnet und voneinander durch das mit der Schnalle 22 zusammenwirkende Befestigungselement beabstandet. Nachfolgend wird unter Verweise auf Figur 2 näher auf das Vorsprungelement 34 eingegangen, die diesbezüglichen Ausführungen sind unmittelbar auf das Vorsprungelement 35 anzuwenden. Die Vorsprungelemente 36 und 37 unterscheiden sich nur in geringem Ausmaß vom Vorsprungelement 34, so dass eine gesonderte Erläuterung der Vorsprungelemente 36 und 37 nicht erfolgt.

Beim Vorsprungelement 34 handelt es sich um eine von der Seitenwand 16 und insbesondere einstückig mit dieser gebildete parallel zur Bodenwand 14 und zum Deckel 20 verlaufende horizontale Sicke 38. Die Sicke 38 springt von der Seitenwand 16 im Übrigen in Richtung der Außenseite des Sterilbehälters 10 hervor, und zwar bis zum Deckelrand 24. "Hervorspringen von der Seitenwand 16" bedeutet vorliegend, dass das Vorsprungelement 34 von der Seitenwand 16 gegenüber einem Verlauf der Seitenwand 16 hervorspringt, den diese von der Bodenwand 14 bis zum freien Rand 18 aufwiese, wäre das Vorsprungelement 34 nicht vorhanden.

Die Sicke 38 ist ungefähr etwas oberhalb der Mitte der Seitenwand 16 angeordnet. Unterhalb der Sicke 38 weist die Seitenwand 16 einen unteren Wandbereich 40 bis zur Bodenwand 14 auf, und oberhalb der Sicke 38 weist die Seitenwand 16 einen oberen Wandbereich 42 bis zum freien Rand 18 auf. Die Seitenwand 16 ist so geformt, dass der obere Wandbereich 42 in Richtung einer Sterilbehältermitte des Sterilbehälters 10 nicht über den unteren Wandbereich 40 hervorragt, sondern sogar hinter diesem zurückbleibt. Der obere Wandbereich 42 ragt daher bezüglich des unteren Wandbereiches 40 in Richtung einer Außenseite des Sterilbehälters 10 hervor. Dies bedeutet, dass sich der Behälterinnenraum 28 in Richtung des freien Randes 18 erweitert.

Wie erwähnt, steht die Sicke 38 bis zum Deckelrand 24 von der Seitenwand 16 hervor. Dabei bildet sie obenseitig ein Anlageelement 44 für ein unteres Ende des Deckelrandes 24. Ist der Sterilbehälter 10 geschlossen, kann sich der Deckelrand 24 mit dem unteren Ende auf dem Anlageelement abstützen.

Der Sterilbehälter 10 weist folgende Vorteile auf:
Da sich der Deckelrand 24 im geschlossenen Zustand auf der Sicke 38 abstützen kann, ist es möglich, den Deckel 20 mit einer höheren Zugkraft an der Wanne 12 zu befestigen, als dies ohne Abstützen möglich wäre. Dies führt dazu, dass im Bereich des Dichtelementes 26 ein unerwünschter Gasaustausch vom Behälterinnenraum zur Atmosphäre durch einen Spalt zwischen dem freien Rand 18 und dem Deckel 20 hindurch nur mit äußerst geringer Wahrscheinlichkeit erfolgen kann. Stattdessen erfolgt der Gasaustausch bestimmungsgemäß durch die Filtereinrichtungen 30 und 32 hindurch, was insgesamt das Sterilisationsergebnis unter Einsatz des Sterilbehälters 10 verbessert.

Zudem schützt die Sicke 38 den Deckelrand 24 gegen unbeabsichtigtes Anstoßen oder Untergreifen des Deckels 20 in einer von der Wanne 12 weg weisenden Richtung. Dadurch ist die Wahrscheinlichkeit verringerbar, dass sich trotz der verbesserten Abdichtung am Dichtelement 26 ein Spalt zwischen dem freien Rand 18 und dem Deckel 20 ausbilden kann, durch welchen hindurch ein Gasaustausch vom Behälterinnenraum 28 in die Atmosphäre und umgekehrt möglich ist. Auch dies wirkt sich vorteilhaft auf das Sterilisationsergebnis aus. Im Falle eines solchen Gasaustausches könnte es nämlich erforderlich sein, einen neuen Sterilisationsvorgang durchführen zu müssen, um Keime abzutöten, welche zwischen dem freien Rand 18 und dem Deckel 20 in den Behälterinnenraum 28 gelangen können und darin zur Sterilisation bestimmte chirurgische Instrumente kontaminieren. Dies verbessert somit auch die Handhabung des Sterilbehälters 10.

Außerdem wird dessen Handhabbarkeit dadurch verbessert, dass der obere Wandbereich 42, wie erwähnt, bezüglich des unteren Wandbereiches 40 in Richtung einer Außenseite des Sterilbehälters 10 vorspringt, so dass sich der Behälterinnenraum 28 in Richtung des freien Randes 18 erweitert. Dies erleichtert es der Bedienperson, chirurgische Instrumente und/oder Siebkörbe mit chirurgischen Instrumenten in den Behälterinnenraum 28 einzuführen oder aus diesem zu entnehmen.

Die Wahrscheinlichkeit, dass eine Bedienperson beim Einführen oder Entnehmen der Instrumente an der Seitenwand 16 anstößt oder hängen bleibt, ist äußerst gering. Diese Instrumente können der Bedienperson dadurch auch nicht aus der Hand gleiten und zu Boden fallen, wodurch das Sterilisationsergebnis zunichte gemacht werden könnte. In entsprechender Weise ist die Wahrscheinlichkeit äußerst gering, dass eine Bedienperson beim Entnehmen eines mit Instrumenten gefüllten Siebkorbes an der Seitenwand 16 anstößt oder hängen bleibt, was schlimmstenfalls zum Herausschleudern eines Instrumentes aus dem Siebkorb und zu dessen Kontaminierung führen könnte. Somit erweist sich die Tatsache, dass der obere Wandbereich 42 bezüglich des unteren Wandbereiches 40 in Richtung einer Sterilbehältermitte nicht hervorragt, sondern sogar zurückspringt, ebenfalls als vorteilhaft zur Erzielung eines möglichst guten Sterilisationsergebnisses.

Die einstückig mit der Seitenwand 16 gebildete Sicke 38 ist auf konstruktiv einfache Weise herstellbar, und zudem versteift sie die Seitenwand 16, so dass der Sterilbehälter 10 eine hohe Formstabilität aufweist.

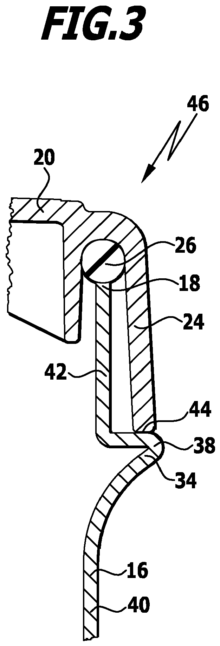

Bei einer Variante des Sterilbehälters 10 kann vorgesehen sein, dass der Deckelrand 24 ist so bemessen ist oder dass die Sicke 38 an der Seitenwand 16 derart angeordnet ist, dass der Deckelrand 24 im geschlossenen Zustand des Sterilbehälters 10 von der Sicke 38 beabstandet ist und die Sicke 38 kein Anlageelement ausbildet. Dies begünstigt es, dass ein Überdruck im Inneren des Sterilbehälters 10 auch dadurch abgebaut werden kann, dass sich der Deckel 20 in Folge des Überdrucks leicht verformt und/oder etwas von der Seitenwand 16 angehoben wird unter Freigabe eines Nebenstrompfades zwischen dem Deckel 20 und der Seitenwand 16. In entsprechender Weise kann vorgesehen sein, dass bei Varianten der nachfolgend erläuterten Ausführungsformen 46, 48, 50, 52, 54, 56, 60, 66 und 68 gemäß den Figuren 3 bis 11 des Sterilbehälters der Deckelrand 24 relativ zum jeweiligen Vorsprungelement des Sterilbehälters beabstandet ist.

In den Figuren 3, 4, 5, 6 und 7 sind weitere bevorzugte Ausführungsformen 46, 48, 50, 52 bzw. 54 des Sterilbehälters in einer dem Detail A in Figur 2 entsprechenden Darstellung teilweise gezeigt. Vom Sterilbehälter 10 unterscheiden sich die Sterilbehälter 46 bis 54 jeweils allein dadurch, wie die Sicke 38 der Seitenwand 16 gebildet ist. In den Figuren 3 bis 7 werden daher für die Merkmale und Bauteile des Sterilbehälters 10 und der Sterilbehälter 46 bis 54 jeweils identische Bezugszeichen benutzt. Die mit dem Sterilbehälter 10 erzielbaren Vorteile können mit den Sterilbehältern 46 bis 54 ebenfalls erzielt werden.

Beim Sterilbehälter 46 gemäß Figur 3 ist die Sicke 38 hakenförmig gebildet. Ihr Anlageelement 44 ist an einem horizontalen Absatz für ein zuverlässiges Aufliegen des Deckelrandes 24 gebildet.

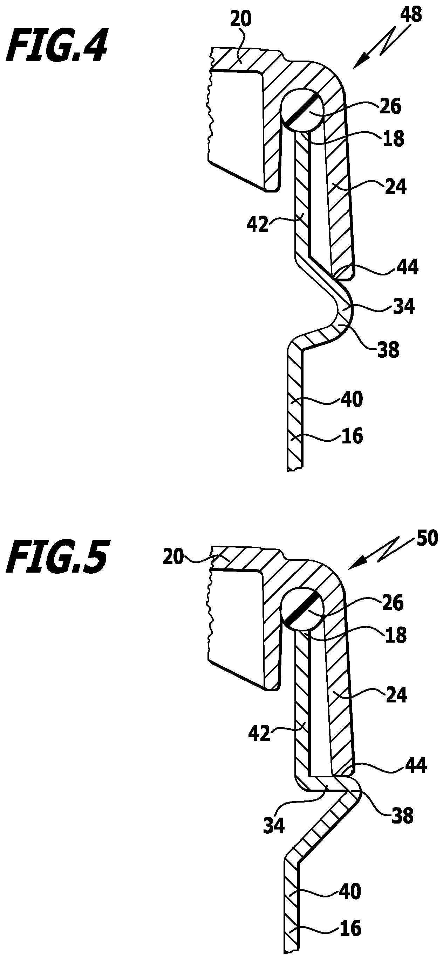

Beim Sterilbehälter 48 gemäß Figur 4 ist die Sicke 38 näherungsweise bogenförmig gebildet und konstruktiv einfach gefertigt.

Beim Sterilbehälter 50 gemäß Figur 5 ist die Sicke 38 dreieckförmig und insbesondere sägezahnförmig gebildet. Sie bildet ebenfalls einen horizontalen Absatz mit Anlageelement 44 für den Deckelrand 24.

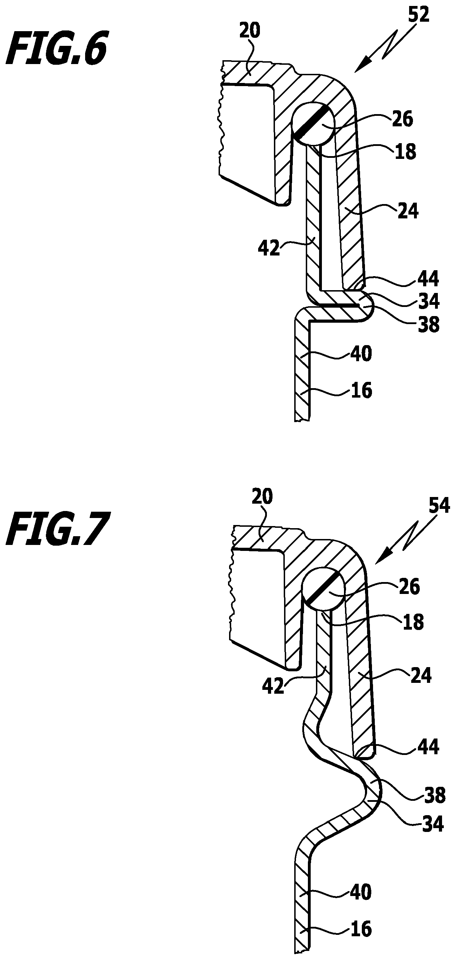

Beim Sterilbehälter 52 gemäß Figur 6 ist die Sicke 38 pulsförmig gebildet, und sie weist einen horizontalen Absatz mit Anlageelement 44 für den Deckelrand 24. Ferner verleiht sie der Seitenwand 16 eine hohe Steifigkeit.

Beim Sterilbehälter 54 gemäß Figur 7 ist die Sicke 38 wellenförmig, d.h. in Gestalt eines Wellenzuges gebildet, wodurch sie der Seitenwand 16 eine hohe Steifigkeit verleiht.

Bei den Sterilbehältern 46 bis 54 ist die Sicke 38 jeweils eine einstückig mit der Seitenwand 16 gebildete Vollsicke.

Die Sicken 38 der Sterilbehälter 46, 50, 52 und 54 ragen außenseitig über den Deckelrand 24 hinaus und schützen ihn besonders wirkungsvoll gegenüber Anstoßen und/oder Untergreifen.

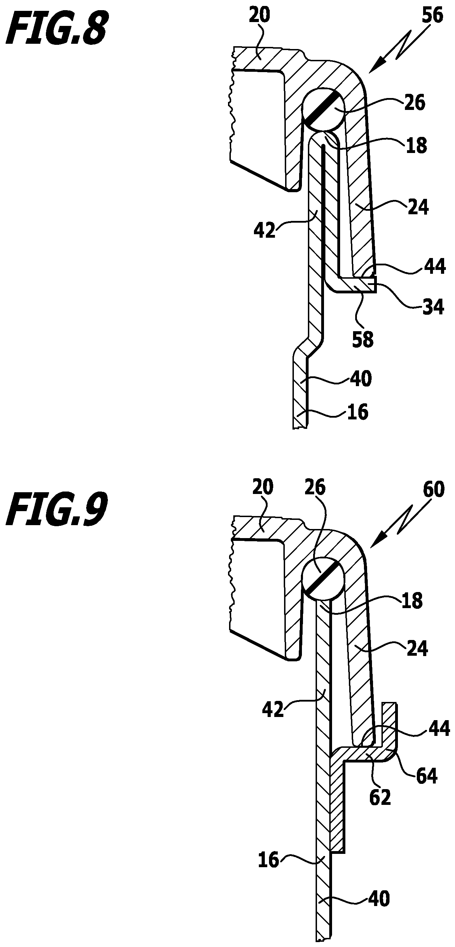

Figur 8 zeigt eine bevorzugte Ausführungsform 56 des erfindungsgemäßen Sterilbehälters, dessen Vorsprungelement 34 einstückig mit der Seitenwand 16 gebildet ist. Im Fall des Sterilbehälters 56 ist die Seitenwand 16 am freien Rand 18 um 180° in Richtung der Unterseite des Sterilbehälters 56 umgebogen, und zwar außenseitig am Sterilbehälter 56. Dadurch ist der obere Wandbereich 42 doppellagig ausgebildet. Im Abstand zum freien Rand 18 ist die Seitenwand 16 in Richtung der Außenseite des Sterilbehälters 56 erneut umgebogen, so dass ein horizontaler Absatz 58 entsteht, der das Vorsprungelement 34 des Sterilbehälters 56 bildet.

Die auf vorstehend beschriebene Weise umgebogene Seitenwand 16 des Sterilbehälters 56 weist eine besonders hohe Steifheit auf. Was die mit dem Sterilbehälter 56 erzielbaren Vorteile betrifft, kann auf obenstehende Erläuterungen bezüglich der Sterilbehälter 10 und 46 verwiesen werden.

Eine in Figur 9 dargestellte weitere bevorzugte Ausführungsform 60 eines Sterilbehälters umfasst ein Vorsprungelement 62 in Gestalt eines im Querschnitt stufenförmigen und längserstreckten, (d.h. senkrecht zur Zeichenebene verlaufenden) Profils 64. Das Profil 64 ist an der beim Sterilbehälter 60 planar ausgestalteten Seitenwand 16 unlösbar festgelegt, beispielsweise durch Kleben, Schweißen, Nieten oder Verschrauben. Es kann, da getrennt vom Sterilbehälter 60 hergestellt, aus einem andersartigen Material gefertigt sein. Dadurch lassen sich unter Umständen Herstellungskosten des Sterilbehälters 60 gegenüber denjenigen des Sterilbehälters 10 einsparen.

Anstelle des Profils 64 könnte bei dem Sterilbehälter 60 ein Profil zum Einsatz kommen, welches lösbar an der Seitenwand 16 festgelegt werden kann, beispielsweise mittels einer Rast-, Klemm- oder Steckverbindung.

Das Profil 64 ragt von der planaren Seitenwand 16 in Richtung der Außenseite des Sterilbehälters 60 so über den Deckelrand 24 hinaus, so dass es diesen auf der der Seitenwand 16 abgewandten Seite hintergreift. Dadurch ist der Deckelrand 24 auf besonders zuverlässige Weise durch Anstoßen und/oder Untergreifen durch eine Bedienperson geschützt.

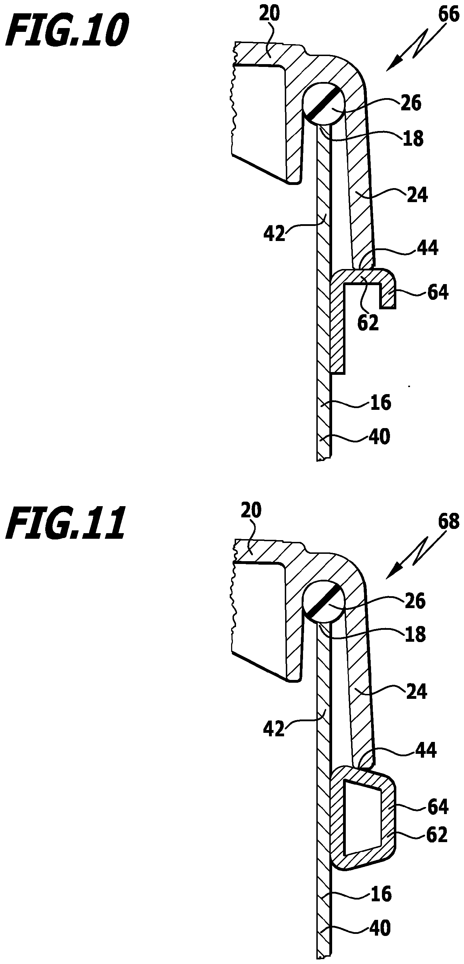

In entsprechender Weise wie der Sterilbehälter 60 umfassen in den Figuren 10 und 11 dargestellte bevorzugte Ausführungsformen 66 bzw. 68 des Sterilbehälters jeweils ein getrennt von der Seitenwand 16 gefertigtes und an dieser festgelegtes Profil, das außenseitig über den Deckelrand 24 hervorspringt. Für das jeweilige Profil der Sterilbehälter 66 und 68 sowie das es jeweils bildende Vorsprungelement werden dieselben Bezugszeichen benutzt wie im Falle des Sterilbehälters 60.

Beim Sterilbehälter 66 gemäß Figur 10 ist das Profil 64 J-förmig ausgebildet.

Beim Sterilbehälter 68 gemäß Figur 11 ist das Profil 64 ein Hohlprofil.

Die mit dem Sterilbehälter 10 vorstehend erläuterten erzielbaren Vorteile können bei den Sterilbehältern 60, 66 und 68 ebenfalls erzielt werden, so dass diesbezüglich auf obenstehende Erläuterungen verwiesen werden kann.

## Patentansprüche

1. Sterilbehälter (56) zur Sterilisation chirurgischer Instrumente, umfassend eine Bodenwand (14), eine Seitenwand (16) sowie einen Deckel (20), der in einer geschlossenen Stellung des Sterilbehälters (56) mit einem Deckelrand (24) einen an einer der Bodenwand (14) abgewandten Seite der Seitenwand (16) angeordneten freien Rand übergreift, wobei der Sterilbehälter (56) mindestens ein zwischen der Bodenwand (14) und dem freien Rand (18) an der Seitenwand (16) angeordnetes Vorsprungelement (34) umfasst, das von der Seitenwand (16) in Richtung einer Außenseite des Sterilbehälters (56) zumindest bis zum Deckelrand (24) vorspringt und einen geringeren Abstand zur Bodenwand (14) aufweist als der Deckelrand (24), wobei die Seitenwand (16) in einem oberen Wandbereich (42) zwischen dem mindestens einen Vorsprungelement (34) und dem freien Rand (18) nicht in Richtung einer Sterilbehältermitte des Sterilbehälters (56) über die Seitenwand (16) in einem unteren Wandbereich (40) zwischen dem mindestens einen Vorsprungelement (34) und der Bodenwand (14) hervorragt, wobei das mindestens eine Vorsprungelement (34) aus Metall gefertigt und einstückig mit der Seitenwand (16) gebildet ist, **dadurch gekennzeichnet, dass** die Seitenwand (16) am freien Rand (18) um 180° in Richtung der Bodenwand (14) außenseitig umgebogen ist und der obere Wandbereich (42) doppellagig ausgebildet ist und dass die Seitenwand (16) im Abstand zum freien Rand (18) in Richtung der Außenseite umgebogen ist, so dass ein das mindestens eine Vorsprungelement (34) ausbildender Absatz (58) vorhanden ist.

2. Sterilbehälter nach Anspruch 1, **dadurch gekennzeichnet, dass** die Seitenwand (16) im oberen Wandbereich (42) zwischen dem mindestens einen Vorsprungelement (34) und dem freien Rand (18) in Richtung der Außenseite über die Seitenwand (16) im unteren Wandbereich (40) zwischen dem mindestens einen Vorsprungelement (34) und der Bodenwand (14) hervorragt.

3. Sterilbehälter nach Anspruch 1 oder 2, **dadurch gekennzeichnet, dass** mindestens ein Vorsprungelement (34) über den Deckelrand (24) hinaus in Richtung der Außenseite von der Seitenwand (16) vorspringt.

4. Sterilbehälter nach Anspruch 3, **dadurch gekennzeichnet, dass** mindestens ein Vorsprungelement (34) den Deckelrand (24) an einer der Seitenwand (16) abgewandten Seite hintergreift.

5. Sterilbehälter nach einem der voranstehenden Ansprüche, **dadurch gekennzeichnet, dass** mindestens ein Vorsprungelement (34) ein Anlageelement (44) für den Deckelrand (24) umfasst oder ausbildet.

6. Sterilbehälter nach einem der voranstehenden Ansprüche, **dadurch gekennzeichnet, dass** der Absatz (58) ein horizontaler Absatz (58) ist.

7. Sterilbehälter nach einem der voranstehenden Ansprüche, **dadurch gekennzeichnet, dass** mindestens ein Vorsprungelement (34) an einer Längsseite des Sterilbehälters (56) angeordnet ist.

8. Sterilbehälter nach einem der voranstehenden Ansprüche, **dadurch gekennzeichnet, dass** mindestens ein Vorsprungelement (34) an einer Querseite des Sterilbehälters (56) angeordnet ist.

9. Sterilbehälter nach einem der voranstehenden Ansprüche, **dadurch gekennzeichnet, dass** mindestens ein Vorsprungelement (34) längs eines gesamten Umfanges oder im Wesentlichen längs eines gesamten Umfanges des Sterilbehälters (56) verläuft.

10. Sterilbehälter nach einem der voranstehenden Ansprüche, **dadurch gekennzeichnet, dass** mindestens ein Vorsprungelement (34) entlang einer zusammenhängenden Randlinie längs eines Umfanges des Sterilbehälters (56) verläuft.

11. Sterilbehälter nach Anspruch 10, **dadurch gekennzeichnet, dass** eine Mehrzahl von Vorsprungelementen (34), jeweils entlang einer zusammenhängenden Randlinie längs eines Umfangs des Sterilbehälters (56) verlaufen und in Umfangsrichtung voneinander beabstandet sind.

12. Sterilbehälter nach einem der voranstehenden Ansprüche, **dadurch gekennzeichnet, dass** der Sterilbehälter (56) genau ein Vorsprungelement (34) umfasst.

13. Sterilbehälter nach einem der Ansprüche 1 bis 11, **dadurch gekennzeichnet, dass** der Sterilbehälter (56) eine Mehrzahl von Vorsprungelementen (34) umfasst.

14. Sterilbehälter nach Anspruch 13, **dadurch gekennzeichnet, dass** alle Vorsprungelemente (34) des Sterilbehälters (56) identisch ausgebildet sind.

## Claims

1. Sterile container (56) for the sterilization of surgical instruments, comprising a bottom wall (14), a side wall (16) and a lid (20) which, in a closed position of the sterile container (56), with a lid rim (24) engages over a free rim arranged on a side of the side wall (16) that faces away from the bottom wall (14), wherein the sterile container (56) has at least one projection element (34) arranged between the bottom wall (14) and the free rim (18) on the side wall (16), the projection element projecting from the side wall (16) in the direction of an outside of the sterile container (56) at least as far as the lid rim (24) and being located at a shorter distance from the bottom wall (14) than is the lid rim (24), the side wall (16), in an upper wall area (42) between the at least one projection element (34) and the free rim (18), not protruding in the direction of a sterile container center of the sterile container (56) over the side wall (16) in a lower wall area (40) between the at least one projection element (34) and the bottom wall (14), wherein the at least one projection element (34) is made of metal and is formed integrally with the side wall (16), **characterized in that** the side wall (16) is bent over at the free end (18) through 180° in the direction of the bottom wall (14) at the outside and the upper wall area (42) is of double-layered configuration and **in that** at a distance from the free rim (18), the side wall (16) is bent over in the direction of the outside, thereby producing a ledge (58), which forms the projection element (34).

2. Sterile container in accordance with claim 1, **characterized in that** the side wall (16) in the upper wall area (42) between the at least one projection element (34) and the free rim (18) protrudes in the direction of the outside over the side wall (16) in the lower wall area (40) between the at least one projection element (34) and the bottom wall (14).

3. Sterile container in accordance with claim 1 or 2, **characterized in that** at least one projection element (34) projects beyond the lid rim (24) in the direction of the outside from the side wall (16).

4. Sterile container in accordance with claim 3, **characterized in that** at least one projection element (34) engages the lid rim (24) from behind at a side that faces away from the side wall (16).

5. Sterile container in accordance with any one of the preceding claims, **characterized in that** at least one projection element (34) comprises or forms an abutment element (44) for the lid rim (24).

6. Sterile container in accordance with any one of the preceding claims, **characterized in that** the ledge (58) is a horizontal ledge (58).

7. Sterile container in accordance with any one of the preceding claims, **characterized in that** at least one projection element (34) is arranged on a longitudinal side of the sterile container (56).

8. Sterile container in accordance with any one of the preceding claims, **characterized in that** at least one projection element (34) is arranged on a transverse side of the sterile container (56).

9. Sterile container in accordance with any one of the preceding claims, **characterized in that** at least one projection element (34) extends along an entire circumference or substantially along an entire circumference of the sterile container (56).

10. Sterile container in accordance with any one of the preceding claims, **characterized in that** at least one projection element (34) extends along a continuous peripheral line along a circumference of the sterile container (56).

11. Sterile container in accordance with claim 10, **characterized in that** a plurality of projection elements (34) respectively extend along a continuous peripheral line along a circumference of the sterile container (56) and are spaced from one another in the circumferential direction.

12. Sterile container in accordance with any one of the preceding claims, **characterized in that** the sterile container (56) comprises precisely one projection element (34).

13. Sterile container in accordance with any one of claims 1 to 11, **characterized in that** the sterile container (56) comprises a plurality of projection elements (34).

14. Sterile container in accordance with claim 13, **characterized in that** all of the projection elements (34) of the sterile container (56) are of identical construction.

## Revendications

1. Contenant stérile (56), pour la stérilisation d'instruments chirurgicaux, comprenant une paroi de fond (14), une paroi latérale (16) ainsi qu'un couvercle (20) qui, dans une position fermée du contenant stérile (56), surmonte, avec un bord de couvercle (24), un bord libre de la paroi latérale (16) agencé sur le côté de celle-ci opposé à celui où se trouve la paroi de fond (14), contenant stérile (56)
dans lequel le contenant stérile comprend au moins un élément de protubérance (34), qui est agencé sur la paroi latérale (16) entre la paroi de fond (14) et le bord libre (18), fait saillie de la paroi latérale (16) en direction d'un côté extérieur du contenant stérile (56) au moins jusqu'au bord de couvercle (24), et présente une distance à la paroi de fond (14) plus faible que le bord de couvercle (24),
dans lequel la paroi latérale (16), dans une zone de paroi supérieure (42) entre ledit au moins un élément de protubérance (34) et le bord libre (18), ne dépasse pas, en direction d'un centre de contenant stérile dudit contenant stérile (56), de la paroi latérale (16) dans une zone de paroi inférieure (40) entre ledit au moins un élément de protubérance (34) et la paroi de fond (14), et
dans lequel ledit au moins un élément de protubérance (34) est fabriqué en métal et formé d'un seul tenant avec la paroi latérale (16),
**caractérisé en ce que** la paroi latérale (16) est repliée, au niveau du bord libre (18), au côté extérieur, de 180° en direction de la paroi de fond (14), et la zone de paroi supérieure (42) est d'une configuration à double couche, et **en ce que** la paroi latérale (16) est repliée, à distance du bord libre (18), en direction du côté extérieur, de manière à réaliser un gradin (58) formant ledit au moins un élément de protubérance (34).

2. Contenant stérile selon la revendication 1, **caractérisé en ce que** la paroi latérale (16), dans la zone de paroi supérieure (42) entre ledit au moins un élément de protubérance (34) et le bord libre (18), dépasse, en direction du côté extérieur, de la paroi latérale (16) dans la zone de paroi inférieure (40) entre ledit au moins un élément de protubérance (34) et la paroi de fond (14).

3. Contenant stérile selon la revendication 1 ou la revendication 2, **caractérisé en ce qu'**au moins un élément de protubérance (34) fait saillie de la paroi latérale (16) en direction du côté extérieur, au-delà du bord de couvercle (24).

4. Contenant stérile selon la revendication 3, **caractérisé en ce qu'**au moins un élément de protubérance (34) s'engage derrière le bord de couvercle (24) sur le côté opposé à celui dirigé vers la paroi latérale (16).

5. Contenant stérile selon l'une des revendications précédentes, **caractérisé en ce qu'**au moins un élément de protubérance (34) comporte ou forme un élément d'appui (44) pour le bord de couvercle (24).

6. Contenant stérile selon l'une des revendications précédentes, **caractérisé en ce que** le gradin (58) est un gradin horizontal (58).

7. Contenant stérile selon l'une des revendications précédentes, **caractérisé en ce qu'**au moins un élément de protubérance (34) est agencé sur un côté longitudinal du contenant stérile (56).

8. Contenant stérile selon l'une des revendications précédentes, **caractérisé en ce qu'**au moins un élément de protubérance (34) est agencé sur un côté transversal du contenant stérile (56).

9. Contenant stérile selon l'une des revendications précédentes, **caractérisé en ce qu'**au moins un élément de protubérance (34) s'étend le long d'une totalité de la périphérie ou sensiblement le long d'une totalité de la périphérie du contenant stérile (56).

10. Contenant stérile selon l'une des revendications précédentes, **caractérisé en ce qu'**au moins un élément de protubérance (34) s'étend le long d'une ligne de bordure continue le long de la périphérie du contenant stérile (56).

11. Contenant stérile selon la revendication 10, **caractérisé en ce qu'**une pluralité d'éléments de protubérance (34) s'étendent respectivement le long d'une ligne de bordure continue le long de la périphérie du contenant stérile (56), et sont espacés les uns des autres dans la direction périphérique.

12. Contenant stérile selon l'une des revendications précédentes, **caractérisé en ce que** le contenant stérile (56) comporte exactement un élément de protubérance (34).

13. Contenant stérile selon l'une des revendications 1 à 11, **caractérisé en ce que** le contenant stérile (56) comporte une pluralité d'éléments de protubérance (34).

14. Contenant stérile selon la revendication 13, **caractérisé en ce que** tous les éléments de protubérance (34) du contenant stérile (56) sont de configuration identique.
